# EUROPEAN PATENT APPLICATION

(11) **EP 4 800 092 A1**
(43) Date of publication of application: **02.09.2026**
(21) Application number: 25382187.0
(22) Date of filing: 28.02.2025
(51) Int. Cl.: C12M 1/32, C12M 1/00, C12M 1/26, C12M 1/42, C12M 1/02, C12M 1/34

(54) **DEVICE FOR HIGH-THROUGHPUT CONFINEMENT AND IMAGING OF CELLULAR SAMPLES, IN PARTICULAR 3D SAMPLES**

(71) Applicant: European Molecular Biology Laboratory, 69117 Heidelberg (DE)
(72) Inventor: Krauss, Jan B., 08003 Barcelona (ES); Bernardello, Matteo, 08003 Barcelona (ES); Trivedi, Vikas, 08003 Barcelona (ES)
(74) Representative: Krauss, Jan

(57) **Abstract**

The present invention relates to a device (10) for a mechanical confinement of at least one cellular sample (4), comprising a support (1), at least one spacer (2, 2a) of a predefined height and thereby defining a confinement space at the bottom of the support (1), and at least one actuator (3) to be inserted into the support (1), the actuator (3) comprising a surface that provides a confinement of at least one sample (4) positioned in the confinement space. The present invention further relates to an array comprising a multitude of devices (10) according to the invention arranged on a carrier matrix, a method for exerting mechanical confinement to a cellular sample using the device (10) according to the invention, and a method for detecting the effects of mechanical confinement on a cellular sample using the device (10) according to the invention.

## Description

The present invention relates to a device (10) for a mechanical confinement of at least one cellular sample (4), comprising a support (1), at least one spacer (2, 2a) of a predefined height and thereby defining a confinement space at the bottom of the support (1), and at least one actuator (3) to be inserted into the support (1), the actuator (3) comprising a surface that provides a confinement of at least one sample (4) positioned in the confinement space. The present invention further relates to an array comprising a multitude of devices (10) according to the invention arranged on a carrier matrix, a method for exerting mechanical confinement to a cellular sample using the device (10) according to the invention, and a method for detecting the effects of mechanical confinement on a cellular sample using the device (10) according to the invention.

### Background of the invention

Mechanical confinements are needed to change tissue geometry and apply defined forces to biological samples e.g. embryos, 3D organoids, tissue samples. Such studies are typically used to gain insights into the effects of 3D confinement for stem cell and cancer biology, organoid research, biophysics and tissue engineering. Ability to perform such confinements with time-lapse imaging and in a high-throughput fashion is poised to enhance researchers' ability to achieve robust experimental data for such studies.

Prunet A, et al. (in: A new agarose-based microsystem to investigate cell response to prolonged confinement. Lab Chip. 2020 Nov 7;20(21):4016-4030. doi: 10.1039/d0lc00732c. Epub 2020 Sep 25. PMID: 32975276.) disclose that emerging evidence suggests the importance of mechanical stimuli in normal and pathological situations for the control of many critical cellular functions. While the effect of matrix stiffness has been and is still extensively studied, few studies have focused on the role of mechanical stresses. The main limitation of such analyses is the lack of standard in vitro assays enabling extended mechanical stimulation compatible with dynamic biological and biophysical cell characterization. They developed an agarose-based microsystem, the soft cell confiner, which enables the precise control of confinement for single or mixed cell populations. The rigidity of the confiner matches physiological conditions, and its porosity enables passive medium renewal. It is compatible with time-lapse microscopy, in situ immunostaining, and standard molecular analyses, and can be used with both adherent and non-adherent cell lines. Cell proliferation of various cell lines (hematopoietic cells, MCF10A epithelial breast cells and HS27A stromal cells) was followed for several days up to confluence using video-microscopy and further documented by Western blot and immunostaining. Interestingly, even though the nuclear projected area was much larger upon confinement, with many highly deformed nuclei (non-circular shape), cell viability, assessed by live and dead cell staining, was unaffected for up to 8 days in the confiner. However, there was a decrease in cell proliferation upon confinement for all cell lines tested.

The soft cell confiner is therefore said to be a valuable tool to decipher the effects of long-term confinement and deformation on the biology of cell populations. This tool will be instrumental in deciphering the impact of nuclear and cytoskeletal mechanosensitivity in normal and pathological conditions involving highly confined situations, such as those reported upon aging with fibrosis or during cancer.

Malèke Mouelhi et al. (in: Mitosis sets nuclear homeostasis of cancer cells under confinement 2024, eLife 13: RP94975) describe a study, that investigates how a colorectal cancer cell line adapts to prolonged confined environments, with a particular focus on nuclear dynamics under continuous squeezing.

Both publications describe the use of the Agarsqueezer^{®} (Idylle Labs, France), an agarose-based device for squeezing cell culture samples that are in contact with glass on the bottom and agarose on top. It allows very small pre-defined confinement heights (2.5, 5, 30, 100 µm) with tunable agarose stiffness.

The Agarsqueezer^{®}, nevertheless, has several limitations. It comprises many components and requires a long mounting procedure. In a standard multiwell plate size, only two different conditions can be performed, whereas the device according to the invention can handle up to at least 32 samples. The confinement heights are not customizable, and confinement requires manual adjustment by screwing, and so the final confinement is prone to operator-dependent bias. The device requires an additional silicon wafer (sold separately) for each of the desired confinement heights. The device is not compatible with a wide range of 3D samples. Finally, the device is expensive a standard set is 1500 Euro, and each silicon wafer is about 500 Euro.

It is therefore an object of the present invention to provide a device for customizable mechanical confinement of living cells, such as cellular 3D samples for experimentation and imaging, in particular in a high-throughput context. Other objects and advantages will become apparent to the person of skill when further studying the present disclosure.

In a first aspect of the present invention, the present invention solves the above object by providing a device (10) for a mechanical confinement of at least one cellular sample (4). The device according to the invention comprises only three main components, a) a support (1) comprising an essentially flat bottom and essentially vertical walls defining a vessel or chamber, b) at least one spacer (2, 2a) positioned on the bottom inside the support (1), wherein the at least one spacer (2, 2a) is of a predefined height and thereby defines a confinement space at the bottom of the support (1), and c) at least one actuator (3) to be inserted into the support (1). The at least one actuator (3) comprises a surface that provides a confinement of at least one sample (4) positioned in the confinement space by contacting the at least one spacer (2, 2a) positioned on the bottom inside the support (1).

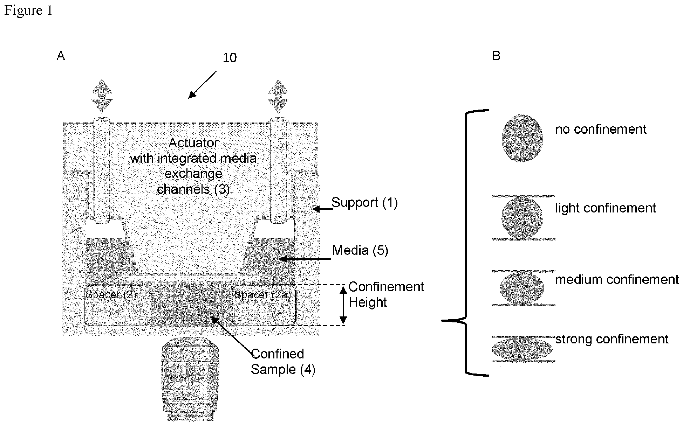

The present invention therefore presents a preferably reusable device for a customizable mechanical confinement of living 3D samples, in particular suitable for high-throughput experimentation and imaging (embodiment see Figure 1). As mentioned, the basic design comprises only three components: (i) the support (1) to define the experimentation space, (ii) spacer(s) (2, 2a) defining the confinement height, and (iii) an actuator (3) to provide the confinement surface, to keep all parts in place and, in a preferred embodiment, to allow media exchange for sample viability and long-term experimentation.

Preferred is the device (10) according to the present invention, wherein the bottom of the support (1) comprises or consists of a material that is permissive for light waves.

More preferred is the device (10) according to the present invention, wherein the vessel or chamber holds a biocompatible polymer, gel or medium, in particular suitable for cell or tissue culture.

Further preferred is the device (10) according to the present invention, wherein the actuator (3) comprises one or more openings or channels to allow the exchange of medium and/or experimental fluids into the vessel or chamber, in particular the confinement space, and/or to allow the insertion of probes and/or sensors into the vessel or chamber, in particular the confinement space.

According to the present invention, the cellular sample (4) that is analyzed or used can be selected from any biological material comprising cells that is suitable to be confined by the device (10) according to the present invention and may be selected from at least one sample comprising one or more of cell lines, such as cancer cell lines or stem cells, organoids and other 3D cultures, embryos and tissue samples, preferably animal cells, such as mammalian cells or tissues, in particular human cells or tissues.

In a second aspect of the present invention, the present invention solves the above object by providing an array comprising a multitude of devices (10) according to the present invention, such as, for example, at least 4, 8, 16, 32, 64 or 128 devices (10) according to the present invention arranged on a carrier matrix, such as a chambered coverslip.

In a third aspect of the present invention, the present invention solves the above object by providing a method for producing the device (10) according to the present invention, comprising the steps of i) providing a suitable support (1) comprising an essentially flat bottom and essentially vertical walls defining a vessel or chamber, such as a chambered coverslip, ii) cutting, in particular laser cutting, of at least one spacer (2, 2a) of a defined height and positioning the at least one spacer on the bottom of the vessel or chamber of the support (1), and iii) producing at least one actuator (3) to be inserted into the support (1) comprising 3D printing or casting thereof, the actuator (3) comprising a surface at the bottom thereof, such a glass coverslip, so that when inserted, the surface at the bottom of the at least one actuator (3) provides a confinement of at least one sample (4) positioned in a confinement space formed by contacting the at least one spacer (2, 2a) positioned on the bottom inside the support (1) with the surface at the bottom of the actuator (3).

In a fourth aspect of the present invention, the present invention solves the above object by providing a method for producing an array comprising a multitude of devices (10) according to the present invention, such as, for example at least 4, 8, 16, 32, 64 or 128 devices (10) according to the present invention, comprising performing the method according to the present invention, wherein a multitude, such as, for example, at least 4, 8, 16, 32, 64 or 128 suitable supports (1) are present or arranged on a suitable carrier matrix, such as a chambered coverslip.

In a fifth aspect of the present invention, the present invention solves the above object by providing a method for exerting mechanical confinement to a cellular sample, comprising providing the device (10) according to the present invention comprising at least one spacer (2, 2a) of a pre-defined height, adding a cellular sample to undergo confinement to the support (1), and inserting the actuator (3) into the support to provide a mechanical confinement to the cellular sample (4).

Preferred is the method according to the present invention, wherein 4, 8, 16, 32, 64 or 128 cellular samples are confined in a high throughput array according to the present invention.

In a sixth aspect of the present invention, the present invention solves the above object by providing a method for detecting the effects of mechanical confinement on a cellular sample, comprising performing the method according to the present invention, and suitably detecting the effects of the confinement using imaging techniques, such as time-lapse imaging, microscopic analysis, detection in the shape or morphology of the cells and/or cellular sample, such as the deformation of cell populations, detecting the impact of nuclear and cytoskeletal mechanosensitivity, and/or the detection of nuclear dynamics under continuous squeezing. Preferred is the method according to the present invention, further comprising the addition of experimental fluids into the vessel or chamber, such as dyes, nutrients, drugs or other bioactive substances.

In a seventh aspect of the present invention, the present invention solves the above object by providing the use of the device according to the present invention or the array according to the present invention for determining the effects of confinement on a cellular sample, such as the effects of long-term confinement on a cellular sample, such as, for example, determining the impact of nuclear and cytoskeletal mechanosensitivity in normal and pathological conditions involving confined environments, such as, for example in conditions selected from aging, fibrosis and cancer.

As mentioned above, in a first aspect thereof, the present invention provides a device (10) for a mechanical confinement of at least one cellular sample (4). The inventive device comprises fewer parts than the devices according to the state of the art, namely a support (1), at least one spacer (2, 2a), and an actuator (3).

The present invention in particular provides a preferably reusable device for a customizable mechanical confinement of living 3D samples, in particular suitable for high-throughput experimentation and imaging (embodiment see Figure 1).

According to the present invention, the support (1) comprises an essentially flat bottom and essentially vertical walls defining a vessel or chamber. Therefore, preferably the support (1) defines the experimentation space or volume in that it represents a container, such as a well or "beaker" holding the components of the assay to be performed under confinement. In one embodiment, the support is part of a chambered coverslip, e.g. comprising wells.

The shape of the vessel, well or chamber as defined by the device (10) according to the present invention can be any suitable shape, and may be controlled, for example, by the shape of the actuator. Thus, the vessel, well or chamber may have a round, rectangular, hexagonal or octagonal base or bottom shape or surface.

Since the usual purpose of the device (10) according to the present invention is to provide both confinement and to analyze the effect(s) thereof at the same time, the walls, and in particular the bottom of the support comprises a material or consists of a material that is permissive for light waves, i.e. radiation in the range of wavelengths between 100 nm and 1 mm, preferably between 400 nm and 700 nm (visible light). Preferably, the walls, and in particular the bottom of the support are made of glass, plastic, PDMS, and in particular essentially of PDMS and glass components. The material is preferably transparent. The material may be suitably coated, e.g. for experimental purposes, and preferably is partially or fully coated on the inside of the vessel or chamber with at least one biocompatible molecule, such as a protein or polymer, that modifies its surface properties, such as PLL-PEG or fibronectin.

Since the support (1) defines the experimentation space or volume, preferred is the device (10) according to the present invention, wherein the vessel or chamber holds a biocompatible buffer, polymer, gel or medium, in particular suitable for cell or tissue culture. The choice regarding the medium depends on the nature of the cellular sample, the experiment to be conducted, the duration of the experiment, and analysis method, for example using imaging, microscopic imaging, and/or electrodes or other sensors.

In one embodiment of the device (10) according to the present invention, the bottom and/or the walls of the vessel or chamber include or comprise integrated electrical wires for heating the vessel or chamber, or for electrical stimulation of the samples. This is useful to trigger or accelerate desired reactions in the experimental space, or for a cell culture in the device (10). Furthermore, in another embodiments, the control of the wires, for example in an array (see below), allows to have different temperatures and/or electrical stimulations in the different devices (10), even in one experimental run.

As mentioned above, the at least one spacer (2, 2a) controls the height, and depending on the layout thereof also the final volume, of the experimental space, and the confinement as exerted to the cellular sample. In a preferred device (10) according to the present invention, the at least one spacer (2, 2a) abuts to all or a part of the bottom and all or a part of the walls of the vessel or chamber when defining the confinement space and the experimental space. The spacer (2, 2a) may have a circular, ring, or doughnut shape with the experimental space in the middle. Furthermore, spacers (2, 2a) can be inserted as halves, forming a circular (for example having an indentation), ring, or doughnut shape when assembled. The spacers (2, 2a) may have ends that allow to connect with the neighboring piece. The spacer (2, 2a) may follow the shape of the vessel, and may have a round, rectangular, hexagonal or octagonal outside shape (see also above). Preferred is the device (10) according to the present invention, wherein the at least one spacer (2, 2a) consists of one, two, three, four, or more parts, such as six or eight, arranged as opposite and/or parallel spacers at the bottom of the vessel or chamber. The spacers (2, 2a) are preferably laser-cut to provide any size and shape, any may also be used to add lateral confinement to the otherwise vertical confinement as provided by the flat surface.

With respect to the height (or thickness) of the spacer (2, 2a), this controls the confinement of the cellular sample. The spacers (2, 2a) may have an irregular height or thickness (e.g. may be tapered) in order to, for example, create an indentation, like a round bottom experimental space, or to create an edge that fits with the surface of the actuator (3) as described below. Preferred is the device (10) according to the present invention, wherein the height or thickness of the at least one spacer (2, 2a) at the thickest point thereof measured from the bottom of the support (1) is selected from between 20 µm and 500 µm, preferably between 50 µm and 200 µm.

As mentioned above, the third essential part of the device (10) according to the present invention is the actuator (3). Preferably, the actuator (3) is essentially shaped like a plug or piston to be inserted into the support (1), and preferably closes and/or seals the upper opening of the vessel or chamber upon insertion. The actuator (3) ideally provides a tight seal in order to avoid the uncontrolled evaporation of liquids from the experimental space and the sample, and also avoids the entry of contamination, dirt and dust.

Preferred is the device (10) according to present invention, wherein the actuator (3) has an essentially tapered shape from the top to the bottom (i.e. the part inserted into the inside of the support (1)), and preferably further comprises the surface that provides the confinement of the at least one sample (4) as a disk at essentially the bottom of the actuator (3), when inserted. See Figure 1 for an example. The surface that provides the confinement of the at least one sample (4) usually provides a vertical force to the sample, but may be also have a convex or concave shape (for example for creating a shaped confinement with the spacer and bottom), but also may be of staggered, tapered or slanted shape. Preferably, the actuator (3) is composed of one or several pieces, similar to what has been described above for the spacer (2, 2a). The actuator (3) may also comprise "layers" to be positioned into the support (1), for example a disk as the surface that provides the confinement of the at least one sample (4) at the bottom, with a second part on top that forms the plug or piston. Further preferably, the actuator (3) may be adjustable to fit the dimensions of the support (1) vessel or chamber. This may be realized by an elastic fit or an adjusting screw integrated into the plug or halves of the plug or piston with a spacer fit in between.

In a particular preferred embodiment of the device (10) according to the present invention, the actuator (3) comprises one or more openings or channels that allow the introduction and/or exchange of medium and/or experimental fluids into the vessel or chamber, in particular into the confinement space, and/or allow the insertion of probes and/or sensors into the vessel or chamber, in particular the confinement space. In this embodiment, the device (10) and/or the actuator (3) may comprise vents to control the inflow and/or exit of liquids and/or gases into and from the device in particular the confinement space.

The device (10) and the parts or components thereof according to the present invention may be made from any material suitable for the purpose(s) (see also above), and thus may be made of regular autoclavable, plasma-cleaned, sterilizable, and/or temperature stable material, like steel, glass, plastic, PDMS elastomer, and in particular essentially of PDMS elastomer and glass components (see below).

As a particularly preferred material for the device (10) or the components thereof according to the invention, any suitable biocompatible polymer can be used, preferred is Silicone Elastomer, such as, for example polydimethylsiloxane (PDMS) elastomer (see herein). Examples are known to the person of skill, and can be found in the literature (e.g. Okoshi, M., Yoshida, T. Fabrication of Silicone Rubber-Based Biochip for Disinfection Under Deep-UV Light by ArF Excimer Laser-Induced Photodissociation. Electron. Mater. Lett. 17, 68-73 (2021)).

The device (10) according to the present invention is used to provide a confinement to a suitable cellular sample (4). This may be selected from a sample comprising one or more of cell lines, such as cancer cell lines or stem cells, organoids, pescoids, embryos and tissue samples, preferably animal cells, such as mammalian cells or tissues, in particular human cells or tissues, or even mixtures thereof.

The present invention presents a preferably reusable device for a customizable mechanical confinement of living 3D samples, in particular suitable for high-throughput experimentation and imaging (embodiment see Figure 1). As mentioned, the basic design comprises only three components: (i) the support (1) to define the experimentation space, (ii) spacer(s) (2, 2a) defining the confinement height, and (iii) an actuator (3) to provide the confinement surface, to keep all parts in place and, in a preferred embodiment, to allow media exchange for sample viability and long-term experimentation.

Key advantage of the device and it uses are its simple construction with few components to assemble and maintain the setup (see, for example, Figure 1).

The device (10) according to the present invention is easy to be implemented, the supports may be conventional "µ-Slide 8 Well" plates by idibi (Gräfelfing, Germany), in which each well can host one experimental condition. Spacers (2, 2a) may be made of PDMS elastomer, which is commercially available or can be produced on-site. The actuators (3) may also be PDMS elastomer pillars produced on-site through a custom-designed and 3D printed mold. To each pillar's tip, a glass coverslip may be attached as the confinement surface. The cellular samples are inserted between the spacers followed by the glass slide surface-actuators (3) being inserted inside the supports.

The device (10) according to the present invention can also be integrated into a high-throughput layout and environment (see also below). For this, a multitude of "µ-Slide 8 Well"-supports may be combined in order to obtain multiwell plate sized supports to test multiple samples and/or conditions, while maintaining compatibility with standard microscopic analysis. Also, combinations of cellular samples can fit per support vessel or container.

The system according to the present invention is versatile and customizable, and may be used on multiple 3D samples: cell cultures, embryos (non-human), pescoids, organoids, and 3D cell aggregates, and preferably to confine adherent and non-adherent cells, including human, murine, plant and 3D cell cultures. Spacers (2, 2a) of different confinement heights can be used, and multiple confinement height can be implemented in the same device. The spacers (2, 2a) are preferably laser-cut to provide any size and shape, any may also be used to add lateral confinement to the vertical confinement as provided by the surface.

The support (1) and actuator's (3) surface may be both glass, so they can be plasma-cleaned and coated for clean, sterile, and varying boundary conditions (e.g., adhesive and non-adhesive options).

Media insertion channels may be provided in the actuator (3) for chemical experimentation on the samples.

The system according to the present invention is compatible with long-term and imaging experiments, makes media flow possible in order to sustain samples' development for long-term experiments.

The preferred support (1) is compatible with standard inverted microscopy technology, with any imaging mode: phase-contrast, fluorescence, confocal, etc.

Figure 1 provides a schematic representation of a preferred example of the inventive 3D sample confiner. The embodiment as depicted is designed to be compatible with commercially available 8-well glass-bottom Ibidi plates (1) (gray, support), but a similar procedure could be adapted for other supports as well. PDMS elastomer spacers (2, 2a) (dark gray) are placed on the glass bottom of the plate, which is well-suited for confocal microscopy and can be coated with different molecules to modify surface properties - for example, PLL-PEG for non-adhesive substrates or fibronectin for adhesive/sticky substrates. These spacers (2, 2a) can be laser-cut from commercially available PDMS elastomer sheets (e.g., available from Limitless Shielding Limited, UK) or fabricated on-site using a spin coater to create thin PDMS elastomer layers. The height of these spacers (2, 2a) determines the level of confinement applied to the samples (Figure 1B).

Once the spacers (2, 2a) are put in place, the wells are filled with a medium (pink) suitable for the specific biological sample (dark gray). The system is highly biocompatible, as it consists solely of PDMS elastomer and glass components. These parts can be plasma-cleaned or sterilized using, for example, ethanol. Each well can accommodate different sample types. The number of samples per well depends on their size and medium requirements; for instance, up to four zebrafish blastula explants can be placed within a single well. Their size allows them to place them next to each other, and they survive perfectly within the provided medium for the duration of the experiment (several days). Therefore, in these conditions, a total of 32 samples can be inserted in the setup for one plate. Expanding the setup to 4 plates results in 128 samples that can be imaged at the same time and results in a high-throughput setup.

The sample is positioned in the medium between the spacers (2, 2a), and a top coverslip (light gray) is gently placed on the spacers (2, 2a) to confine it. Finally, a PDMS elastomer actuator (3) (light gray) is positioned on top of the coverslip. This actuator (3) ensures stability by preventing any lateral movement of the coverslip without applying additional force or bending. It is precisely designed to fit within the Ibidi plate. The actuator (3) can be produced on-site using a custom 3D-printed mold. Additionally, small perforations (arrows) in the top allow for medium exchange during long-term imaging and the addition of chemical compounds throughout experiments.

The inventors have specifically designed this embodiment for compatibility with Ibidi 8-well glass-bottom plates, but the system can be adapted to other supports (1) as well. To establish the confinement structure, PDMS elastomer spacers (2, 2a) are laser-cut into precise rectangular shapes and positioned within each well. The PDMS elastomer spacers (2, 2a) can be either commercially purchased or custom-fabricated using a spin coater. Each well (1) contains two spacers (2, 2a), which jointly define the confinement height for the sample.

Following spacer (2, 2a) placement, the wells (1) are filled with an appropriate culture medium, and the biological samples are carefully positioned between the spacers (2, 2a). A top coverslip is then placed over the sample to ensure proper confinement. Finally, a PDMS elastomer actuator (3), fabricated using a custom-designed 3D-printed negative mold, is positioned on top. This actuator (3) is designed to fit precisely within the Ibidi 8-well plate, securing all components without applying additional mechanical stress to the sample.

As mentioned above, the device (10) according to the present invention can also be integrated into a high-throughput layout and environment. For this, for example, a multitude of "µ-Slide 8 Well"-supports may be combined in order to obtain multiwell plate sized supports to test multiple samples and/or conditions, while maintaining compatibility with standard microscopic analysis. Also, combinations of cellular samples can fit per support vessel or container.

Therefore, another preferred embodiment of the invention relates to an array comprising at least 4, 8, 16, 32, 64 or 128 devices (10) according to the present invention arranged on a carrier matrix, such as a chambered coverslip.

Another aspect of the invention then relates to a method for producing the device (10) according to the present invention. The method generally comprises the steps of i) providing a suitable support (1) comprising an essentially flat bottom and essentially vertical walls defining a vessel or chamber, such as a chambered coverslip, ii) laser or otherwise suitably cutting of at least one spacer (2, 2a) of a defined height and positioning the at least one spacer on the bottom of the vessel or chamber of the support (1), and iii) producing at least one actuator (3) to be inserted into the support (1) comprising 3D printing or casting thereof, the actuator (3) comprising a surface at the bottom thereof, such a glass coverslip. When inserted, the surface at the bottom of the at least one actuator (3) provides a confinement of at least one sample (4) positioned in a confinement space formed by contacting the at least one spacer (2, 2a) positioned on the bottom inside the support (1) with the surface at the bottom of the actuator (3).

Another aspect of the invention then relates to a method for producing an array comprising at least 4, 8, 16, 32, 64 or 128 devices (10) according to the present invention, comprising performing the method according to the present invention as above, wherein at least 4, 8, 16, 32, 64 or 128 suitable supports (1) are present or arranged on a suitable carrier matrix, such as a chambered coverslip.

Another aspect of the invention then relates to a method for exerting mechanical confinement to a cellular sample, comprising providing the device (10) according to the present invention comprising at least one spacer (2, 2a) of a pre-defined height, adding a cellular sample to undergo confinement to the support (1), and inserting the actuator (3) into the support to provide a mechanical confinement to the cellular sample (4).

Preferred is the method according to the present invention, wherein, as also mentioned above, the cellular sample (4) is selected from a sample comprising one or more of cell lines, such as cancer cell lines or stem cells, organoids, pescoids, embryos and tissue samples, preferably animal cells, such as mammalian cells or tissues, in particular human cells or tissues, or even mixtures thereof.

The method according to the present invention may also be performed in high-throughput, wherein for example 4, 8, 16, 32, 64 or 128 cellular samples are confined in an array according to the present invention.

Another aspect of the invention then relates to a method for detecting the effects of mechanical confinement on a cellular sample, comprising performing the method according to the present invention, and suitably detecting the effects of the confinement using imaging techniques, such as time-lapse imaging, microscopic analysis, detection in the shape or morphology of the cells and/or cellular sample, such as the deformation of cell populations, detecting the impact of nuclear and cytoskeletal mechanosensitivity, and/or the detection of nuclear dynamics under continuous squeezing. As mentioned, the device is particularly compatible with standard inverted microscopy technology, with any imaging mode: phase-contrast, fluorescence, confocal, etc.

Preferred is the method according to the present invention, further comprising the addition of experimental fluids into the vessel or chamber, such as dyes, nutrients, drugs or other bioactive substances as described herein.

Another aspect of the invention then relates to the use of the device (10) according to the present invention or the array according to the present invention for determining the effects of confinement on a cellular sample, such as the effects of long-term confinement on a cellular sample, such as, for example, determining the impact of nuclear and cytoskeletal mechanosensitivity in normal and pathological conditions involving confined environments, such as, for example in conditions selected from aging, fibrosis and cancer, and for live imaging, for example, for studies of cell viability, proliferation, migration, morphology, nucleus deformability, and cytoskeleton reorganization. Respective test and assays are described herein, and are known in the art.

In the context of the present invention, the term "about" shall mean a deviation of +/- 10% from a given value, unless indicated otherwise.

The present invention relates to the following items:
Item 1. A device (10) for a mechanical confinement of at least one cellular sample (4), comprising a) a support (1) comprising an essentially flat bottom and essentially vertical walls defining a vessel or chamber, b) at least one spacer (2, 2a) positioned on the bottom inside the support (1), wherein the at least one spacer (2, 2a) is of a predefined height and thereby defines a confinement space at the bottom of the support (1), c) at least one actuator (3) to be inserted into the support (1), the at least one actuator (3) comprising a surface that provides a confinement of at least one sample (4) positioned in the confinement space by contacting the at least one spacer (2, 2a) positioned on the bottom inside the support (1).
Item 2. The device (10) according to Item 1, wherein the support defines a vessel or chamber having a round, rectangular, hexagonal or octagonal base or bottom.
Item 3. The device (10) according to Item 1 or 2, wherein the support defines an experimentation space or volume.
Item 4. The device (10) according to any one of Items 1 to 3, wherein the bottom of the support comprises a material that is permissive for light waves, in particular transparent, and preferably is partially or fully coated on the inside of the vessel or chamber with at least one molecule that modifies its surface properties, such as PLL-PEG or fibronectin.
Item 5. The device (10) according to any one of Items 1 to 4, wherein the vessel or chamber holds a biocompatible polymer, gel or medium, in particular suitable for cell or tissue culture.
Item 6. The device (10) according to any one of Items 1 to 5, wherein the bottom and/or the walls of the vessel or chamber comprise integrated electrical wires for heating the vessel or chamber, or for performing electrostimulation tests on the samples.
Item 7. The device (10) according to any one of Items 1 to 6, wherein the at least one spacer (2, 2a) abuts to all or a part of the bottom and all or a part of the walls of the vessel or chamber when defining the confinement space, and in particular has a round, rectangular, hexagonal or octagonal outside shape.
Item 8. The device (10) according to any one of Items 1 to 7, wherein the at least one spacer (2, 2a) consists of one, two, three, four, or more parts, such as six or eight, arranged as opposite and/or parallel spacers at the bottom of the vessel or chamber.
Item 9. The device (10) according to any one of Items 1 to 8, wherein the height or thickness of the at least one spacer (2, 2a) is selected from between 20 µm and 500 µm, preferably between 50 µm and 200 µm.
Item 10. The device (10) according to any one of Items 1 to 9, wherein the actuator (3) is essentially shaped like a plug or piston to be inserted into the support (1), and preferably closes and/or seals the upper opening of the vessel or chamber upon insertion.
Item 11. The device (10) according to any one of Items 1 to 10, wherein the actuator (3) has an essentially tapered shape from the top to the bottom, and preferably further comprises the surface that provides the confinement of the at least one sample (4) as a disk at essentially the bottom of the actuator (3), when inserted.
Item 12. The device (10) according to any one of Items 1 to 11, wherein the actuator (3) is adjustable to fit the dimensions of the support (1) vessel or chamber.
Item 13. The device (10) according to any one of Items 1 to 12, wherein the actuator (3) is composed of one or several pieces.
Item 14. The device (10) according to any one of Items 1 to 13, wherein the actuator (3) comprises one or more openings or channels to allow the exchange of medium and/or experimental fluids into the vessel or chamber, in particular the confinement space, and/or to allow the insertion of probes and/or sensors into the vessel or chamber, in particular the confinement space.
Item 15. The device (10) according to any one of Items 1 to 14, wherein the components thereof are made of autoclavable, plasma-cleaned, sterilizable, and/or temperature stable material, like steel, glass, PDMS elastomer, and in particular essentially of PDMS elastomer and glass components.
Item 16. The device (10) according to any one of Items 1 to 15, wherein the cellular sample (4) is selected from a sample comprising one or more of cell lines, such as cancer cell lines or stem cells, organoids, embryos and tissue samples, preferably animal cells, such as mammalian cells or tissues, in particular human cells or tissues.
Item 17. An array comprising at least 4, 8, 16, 32, 64 or 128 devices (10) according to any one of Items 1 to 16 arranged on a carrier matrix, such as a chambered coverslip.
Item 18. A method for producing the device (10) according to any one of Items 1 to 16, comprising the steps of i) providing a suitable support (1) comprising an essentially flat bottom and essentially vertical walls defining a vessel or chamber, such as a chambered coverslip, ii) laser cutting of at least one spacer (2, 2a) of a defined height and positioning the at least one spacer on the bottom of the vessel or chamber of the support (1), and iii) producing at least one actuator (3) to be inserted into the support (1) comprising 3D printing or casting thereof, the actuator (3) comprising a surface at the bottom thereof, such a glass coverslip, so that when inserted, the surface at the bottom of the at least one actuator (3) provides a confinement of at least one sample (4) positioned in a confinement space formed by contacting the at least one spacer (2, 2a) positioned on the bottom inside the support (1) with the surface at the bottom of the actuator (3).
Item 19. A method for producing an array comprising at least 4, 8, 16, 32, 64 or 128 devices (10) according to any one of Items 1 to 16, comprising performing the method according to Item 18, wherein at least 4, 8, 16, 32, 64 or 128 suitable supports (1) are present or arranged on a suitable carrier matrix, such as a chambered coverslip.
Item 20. A method for exerting mechanical confinement to a cellular sample, comprising providing the device (10) according to any one of Items 1 to 16 comprising at least one spacer (2, 2a) of a pre-defined height, adding a cellular sample to undergo confinement to the support (1), and inserting the actuator (3) into the support to provide a mechanical confinement to the cellular sample (4).
Item 21. The method according to Item 20, wherein the cellular sample (4) is selected from a sample comprising one or more of cell lines, such as cancer cell lines or stem cells, organoids, embryos and tissue samples, preferably animal cells, such as mammalian cells or tissues, in particular human cells or tissues.
Item 22. The method according to Item 20 or 21, wherein 4, 8, 16, 32, 64 or 128 cellular samples are confined in a high throughput array according to Item 17.
Item 23. A method for detecting the effects of mechanical confinement on a cellular sample, comprising performing the method according to any one of Items 20 to 22, and suitably detecting the effects of the confinement using imaging techniques, such as time-lapse imaging, microscopic analysis, detection in the shape or morphology of the cells and/or cellular sample, such as the deformation of cell populations, detecting the impact of nuclear and cytoskeletal mechanosensitivity, and/or the detection of nuclear dynamics under continuous squeezing.
Item 24. The method according to Item 23, further comprising the addition of experimental fluids into the vessel or chamber, such as dyes, nutrients, drugs or other bioactive substances.
Item 25. Use of the device according to any one of Items 1 to 16 or the array according to Item 17 for determining the effects of confinement on a cellular sample, such as the effects of long-term confinement on a cellular sample, such as, for example, determining the impact of nuclear and cytoskeletal mechanosensitivity in normal and pathological conditions involving confined environments, such as, for example in conditions selected from aging, fibrosis and cancer.

The invention will now be described further in the following examples with reference to the accompanying figures, nevertheless, without being limited thereto. For the purposes of the present invention, all references as cited are incorporated by reference in their entireties.
Figure 1 shows: (A) Sectioned lateral view of the overall design: two spacers are inserted within a support, and the sample is then introduced between them. Finally, an actuator is inserted from the top and will confine the specimen at the height dictated by the spacers. (B) By having spacers of different heights, different experimental conditions can be implemented through the level of confinement.

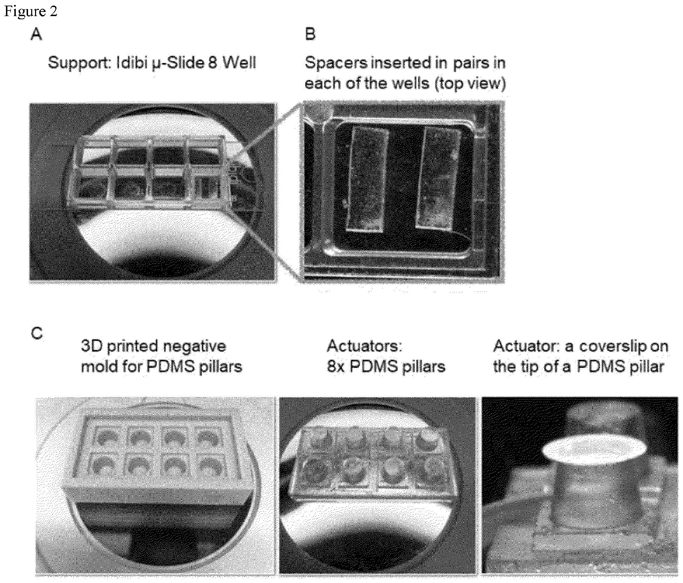

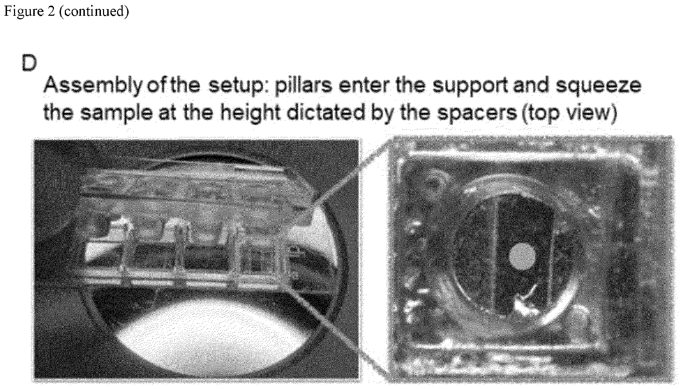

Figure 2 shows: (A) For this embodiment, a commercially available idibi µ-Slide 8 Well can be used as support. (B) Spacers of the defined height are laser cut and inserted in pairs in each of the wells. (C-left) Through a custom-designed and 3D printed negative mold, a set of PDMS elastomer pillars can be generated (C-middle) to be compatible with the idibi µ-Slide 8 Well dimensions. At the tip of each pillar, a glass coverslip is attached (C-right). (D) Samples are inserted between the spacers of each well, and the actuators are positioned from the top. The coverslip will therefore squeeze the samples at the height defined by the spacers.

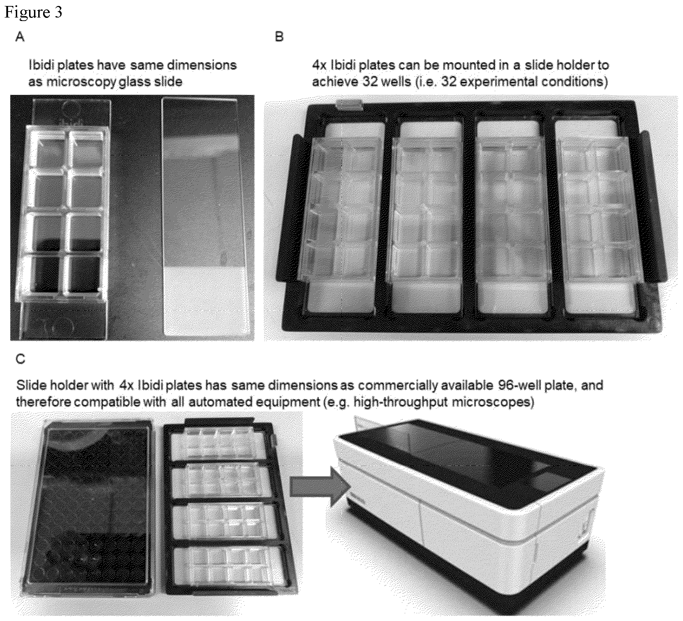

Figure 3 shows that (A) the size of an ibidi plate is identical to a microscopy slide. (B) 4x idibi µ-Slide 8 Well can be mounted together to achieve a standard multiwell plate size, in which up to 32 different conditions can be implemented. In each well, 4 samples can be placed, achieving a total of 32 x 4 = 128 samples. (C) Achieving a multiwell plate standard size means obtaining compatibility with all lab equipment (microscopes, incubators, automated devices).

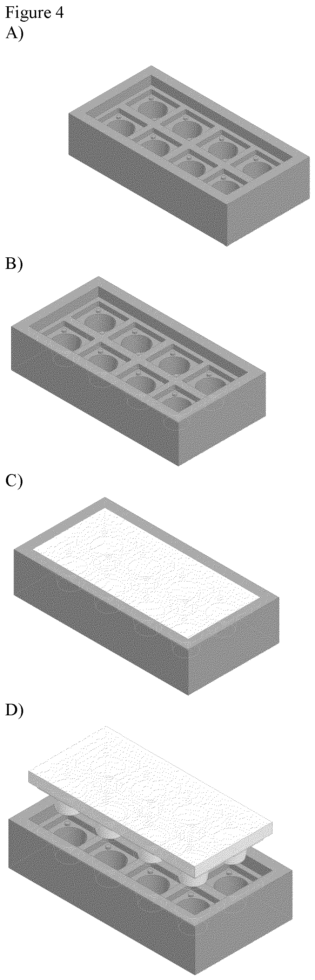

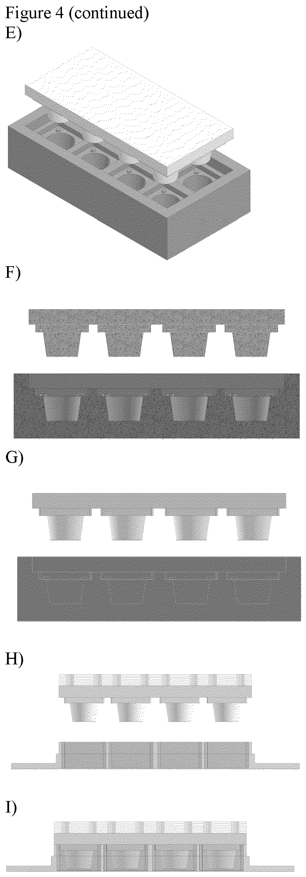

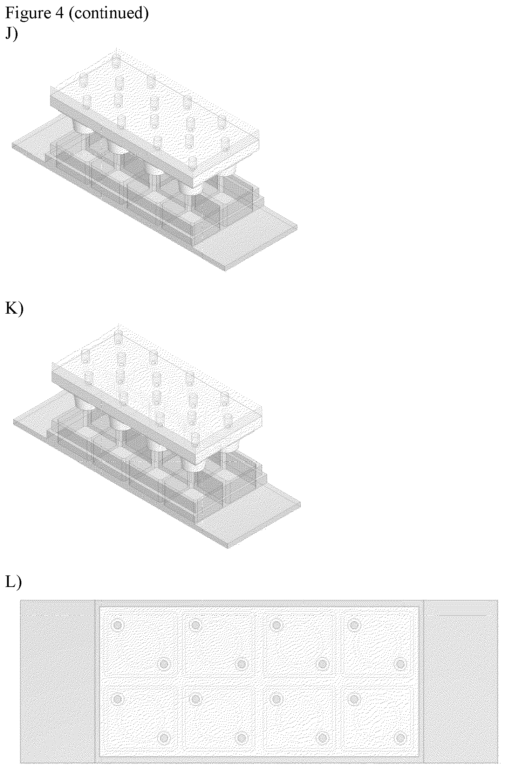

Figure 4 shows an overview of the process of making an embodiment of the device according to the present invention. A) 3D printed mold made from resin as a negative for actuator. B) Small holes are added for perforation holes in the PDMS actuator. C) Resin mold filled. D) Removing the PDMS actuator (beige) from the mold (green). The actuator has all the features of the mold (dotted lines). E) Final structure of PDMS actuator removed from resin mold. F, G) Side section-view of 3D-printed resin mold (green) and PDMS actuator (brown). All solid structures are depicted by dots. H to L) Side-and top views of final structure of PDMS actuator with 4 x 2 rows of columns according to the invention.

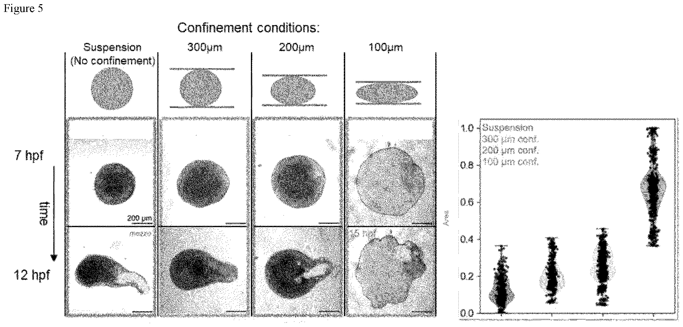

Figure 5 shows 3D cell aggregates developing under different confinement conditions for prolonged time using a device according to the present invention. Aggregates called "pescoids" have been derived from zebrafish embryos and have been squeezed after 7 hours from egg fertilization (hpf=hours post fertilization). Continuing their development under different mechanical constrictions, they result in having different increases in size and different phenotypes.

### Examples

### Materials and methods

### Device fabrication (see Figure 4)

A 3D printed mold was made from resin, which functions as negative for future PDMS elastomer actuator (3). Dimensions in this case were: 57 x 30 x 13 mm. The mold is a negative for the PDMS elastomer actuator (3). The upper part in this embodiment had the same dimensions as an Ibidi 8-well glass bottom plate. The columns were positioned to fit into the wells. Additionally, small holes were added for perforation holes in the PDMS elastomer actuator (3).

The resin mold was filled with PDMS. PDMS was prepared in a 1:18 ratio, mixed well, centrifuged to remove bubbles. After filling the PDMS in the mold, it was incubated in a vacuum chamber for 1 hour to remove further bubbles from the PDMS. The mold containing the PDMS (as in the picture) was baked overnight (~ 16 hours) at 60 °C.

On the next morning, the device was cooled down to room temperature. PDMS remains were removed gently to not damage the mold. Using Isopropanol facilitated removing the PDMS actuator (3, light gray) from the mold (dark gray). A metal spatula with a small tip was used for unmounting the actuator (3). The actuator (3) had all the features of the mold (dotted lines).

Figure 4 shows the final structure of PDMS actuator removed from the resin mold, with a side section-view of 3D-printed resin mold (dark gray) and PDMS actuator (darker gray). All solid structures were filled by dots. Figure 4 also shows a side-view of the final structure of PDMS actuator with 4x2 rows of columns.

The PDMS actuator (3) was attached to a plastic plate for additional stability (clear top part). After sample preparation in an Ibidi 8-well glass bottom plate (gray bottom element), the PDMS elastomer actuator (3) can be placed in the plate. The dimensions of the PDMS elastomer actuator (3) fit seamlessly into the Ibidi 8-well plate.

Figure 4 also shows a diagonal view of lid attached to PDMS actuator (3) over an Ibidi 8-well plate. Perforation holes were added for medium exchange during long-term culturing conditions. Figure 4 also shows a top view of the PDMS elastomer actuator (3) fit into the Ibidi 8-well plate.

### Confinement of organoids

The inventors confined organoids made from zebrafish cells, called pescoids, using spacers of different heights/thicknesses, and cultured these overnight in order to monitor the effects of mechanical confinements (Fig. 5). Pescoids are 3D cell aggregates derived from embryonic cells explanted from the zebrafish embryo, separating them from the extra-embryonic tissue. This separation allows for the in vitro study of the self-organization properties of these cells. The population of pluripotent cells forming the pescoids has the potential to differentiate into all three germ layers of developing embryos, making this in vitro model highly valuable for studying interactions between different cell types during embryonic development. The inventive device (10) was used to manipulate the 3D environment in which pescoids develop and to study its effect on different cell properties. The inventive device (10) was analyzed using time-lapse confocal imaging, which allowed for easy imaging and analysis.

### List of reference numerals

- 1: support
- 2, 2a: spacer(s)
- 3: actuator
- 4: confined sample
- 5: media
- 6:
- 7:
- 8:
- 9:
- 10: confinement device

## Claims

1. A device (10) for a mechanical confinement of at least one cellular sample (4), comprising
a) a support (1) comprising an essentially flat bottom and essentially vertical walls defining a vessel or chamber,
b) at least one spacer (2, 2a) positioned on the bottom inside the support (1), wherein the at least one spacer (2, 2a) is of a predefined height and thereby defines a confinement space at the bottom of the support (1),
c) at least one actuator (3) to be inserted into the support (1), the at least one actuator (3) comprising a surface that provides a confinement of at least one sample (4) positioned in the confinement space by contacting the at least one spacer (2, 2a) positioned on the bottom inside the support (1), wherein preferably the support defines a vessel or chamber having a round, rectangular, hexagonal or octagonal base or bottom.

2. The device (10) according to claim 1, wherein the bottom of the support comprises a material that is permissive for light waves, in particular transparent, and preferably is partially or fully coated on the inside of the vessel or chamber with at least one molecule that modifies its surface properties, such as PLL-PEG or fibronectin.

3. The device (10) according to claim 1 or 2, wherein the vessel or chamber holds a biocompatible polymer, gel or medium, in particular suitable for cell or tissue culture.

4. The device (10) according to any one of claims 1 to 3, wherein the bottom and/or the walls of the vessel or chamber comprise integrated electrical wires for heating the vessel or chamber, or for performing electrostimulation of the samples.

5. The device (10) according to any one of claims 1 to 4, wherein the at least one spacer (2, 2a) abuts to all or a part of the bottom and all or a part of the walls of the vessel or chamber when defining the confinement space, and in particular has a round, rectangular, hexagonal or octagonal outside shape, and/or wherein the at least one spacer (2, 2a) consists of one, two, three, four, or more parts, such as six or eight, arranged as opposite and/or parallel spacers at the bottom of the vessel or chamber.

6. The device (10) according to any one of claims 1 to 5, wherein the height or thickness of the at least one spacer (2, 2a) is selected from between 20 µm and 500 µm, preferably between 50 µm and 200 µm.

7. The device (10) according to any one of claims 1 to 6, wherein the actuator (3) is essentially shaped like a plug or piston to be inserted into the support (1), and preferably closes and/or seals the upper opening of the vessel or chamber upon insertion, and/or wherein the actuator (3) has an essentially tapered shape from the top to the bottom, and preferably further comprises the surface that provides the confinement of the at least one sample (4) as a disk at essentially the bottom of the actuator (3), when inserted.

8. The device (10) according to any one of claims 1 to 7, wherein the actuator (3) is adjustable to fit the dimensions of the support (1) vessel or chamber, and/or wherein the actuator (3) is composed of one or several pieces.

9. The device (10) according to any one of claims 1 to 8, wherein the actuator (3) comprises one or more openings or channels to allow the exchange of medium and/or experimental fluids into the vessel or chamber, in particular the confinement space, and/or to allow the insertion of probes and/or sensors into the vessel or chamber, in particular the confinement space.

10. The device (10) according to any one of claims 1 to 9, wherein the cellular sample (4) is selected from a sample comprising one or more of cell lines, such as cancer cell lines or stem cells, organoids, embryos and tissue samples, preferably animal cells, such as mammalian cells or tissues, in particular human cells or tissues.

11. An array comprising at least 4, 8, 16, 32, 64 or 128 devices (10) according to any one of claims 1 to 10 arranged on a carrier matrix, such as a chambered coverslip.

12. A method for producing the device (10) according to any one of claims 1 to 10, comprising the steps of
i) providing a suitable support (1) comprising an essentially flat bottom and essentially vertical walls defining a vessel or chamber, such as a chambered coverslip,
ii) laser cutting of at least one spacer (2, 2a) of a defined height and positioning the at least one spacer on the bottom of the vessel or chamber of the support (1), and
iii) producing at least one actuator (3) to be inserted into the support (1) comprising 3D printing or casting thereof, the actuator (3) comprising a surface at the bottom thereof, such a glass coverslip,
so that when inserted, the surface at the bottom of the at least one actuator (3) provides a confinement of at least one sample (4) positioned in a confinement space formed by contacting the at least one spacer (2, 2a) positioned on the bottom inside the support (1) with the surface at the bottom of the actuator (3).

13. A method for exerting mechanical confinement to a cellular sample, comprising providing the device (10) according to any one of claims 1 to 10 comprising at least one spacer (2, 2a) of a pre-defined height, adding a cellular sample to undergo confinement to the support (1), and inserting the actuator (3) into the support to provide a mechanical confinement to the cellular sample (4), wherein preferably the cellular sample (4) is selected from a sample comprising one or more of cell lines, such as cancer cell lines or stem cells, organoids, embryos and tissue samples, preferably animal cells, such as mammalian cells or tissues, in particular human cells or tissues, and more preferably wherein 4, 8, 16, 32, 64 or 128 cellular samples are confined in a high throughput array according to claim 11.

14. A method for detecting the effects of mechanical confinement on a cellular sample, comprising performing the method according to claim 13, and suitably detecting the effects of the confinement using imaging techniques, such as time-lapse imaging, microscopic analysis, detection in the shape or morphology of the cells and/or cellular sample, such as the deformation of cell populations, detecting the impact of nuclear and cytoskeletal mechanosensitivity, and/or the detection of nuclear dynamics under continuous squeezing, preferably further comprising the addition of experimental fluids into the vessel or chamber, such as dyes, nutrients, drugs or other bioactive substances.

15. Use of the device according to any one of claims 1 to 10 or the array according to claim 11 for determining the effects of confinement on a cellular sample, such as the effects of long-term confinement on a cellular sample, such as, for example, determining the impact of nuclear and cytoskeletal mechanosensitivity in normal and pathological conditions involving confined environments, such as, for example in conditions selected from aging, fibrosis and cancer
